# EUROPEAN PATENT SPECIFICATION

(11) **EP 2 581 063 B1**
(45) Date of publication and mention of the grant of the patent: **01.10.2014**
(21) Application number: 11275124.3
(22) Date of filing: 14.10.2011
(51) Int. Cl.: A61F 2/50, B29C 33/38, B29C 51/26, A61F 2/00, A61F 2/80

(54) **Preform for making a prosthetic limb socket**
Vorform zur Herstellung eines Schafts für prothetische Gliedmaßen
Préforme de fabrication d'une emboîture de prothèse

(43) Date of publication of application: 17.04.2013
(73) Proprietor: Bulldog Tools, Inc., Lewisburg, OH 45338 (US)
(72) Inventor: Meyer, Robert B, Lewisburg, Ohio 45338 (US); Meyer, Wilbur N., Lewisburg, Ohio 45338 (US)
(74) Representative: Cabinet Chaillot

(56) References cited:
- GB-A- 534 617
- JP-A- 4 176 630
- US-A- 3 466 706
- US-A- 6 050 875
- US-A1- 2002 043 738
- US-B2- 6 551 683

## Description

### Background of the Invention

This invention relates to the production of a substantially rigid plastic socket for receiving the stump or residual limb of a partial amputee, such as, for example, a prosthetic limb socket as disclosed in U.S. Patent No. 5,980,803 and No. 6,551,683. In the forming of a socket in accordance with No. 5,980,803, it is common to extrude a large rectangular flat sheet of thermoplastics material and with a predetermined thickness, such as one-half inch, and then cut the sheet into a plurality of smaller square pieces or sheets, for example, 24 inch square sheets. A peripheral portion of a square sheet is clamped within a square metal clamping frame and the square frame and sheet are placed within an oven having a temperature of about 400°F and until the square plastic sheet softens. The heated square plastic sheet and the attached square frame are then removed from the oven by manually gripping the frame with protective gloves, and the sheet is drawn and stretched downwardly over a hollow positive model of the stump or residual limb.

In accordance with Patent No. 6,551,683, which issued to an inventor of the present invention and discloses a preform according to the preamble of appended claim 1, a metal reinforcing ring is molded within a peripheral portion of an injection molded disk. The positive model is commonly mounted on a vacuum base or pedestal which creates a vacuum within the hollow model and through fine holes or pores within the model while the heated plastic sheet or disk is stretched over the model to form a socket conforming to the model. The positive model of the patient's residual limb is commonly produced by forming a plaster cast on the patient's residual limb, removing the cast after it hardens and filling the cast with a plaster to make a positive model. The cast is then removed or broken away from the positive model with a pneumatic chisel. A positive model may also be made, for example, as disclosed in U.S. Patent No. 5,901,060, that is, by using a digitized impression of the residual limb to machine the model. After a socket is formed, it usually receives a coupler such as the socket coupler disclosed in U.S. Patent No. 6,106,559 which issued to an applicant of the present invention.

It has been determined that the above method for making a socket using a square plastic sheet cut from a larger extruded sheet is slow and expensive and results in producing significant scrap from the sheet, primarily due to the square corner portions of the sheet which are scrapped along with the trimmed base portion of the drawn sheet used to form the socket. Also, the clamping frame for the rectangular sheet is relatively expensive, has a limited service life, and requires significant time to be properly attached to the peripheral portion of the square sheet and then removed from the sheet after the heated sheet is stretched over the positive model. The method of making the square sheet, the corner portions of the square sheet and the significant time required for attaching the clamping frame to the square sheet and removing the frame after forming a socket, add significantly to the cost of producing the socket. While the above method of making a socket with an injection molded disk having an embedded metal reinforcing ring significantly decreases the time required for making a socket, the reinforcing ring adds to the cost of the disk, and the ring is not reusable and becomes scrap along with the base portion of sheet or disk removed from the socket.

GB 534617 discloses a process for moulding nasal inhalers from a thermoplastic material, in which a sheet of thermoplastic material is clamped in a frame in order to prevent relative movement of edges of the sheet during moulding.

US 3466706 discloses a clamping device for securing thermoplastic sheet material during moulding of that material. A picture frame shaped base member contains a clamping member along each of its sides.

### Summary of the Invention

The present invention is directed to an improved preform as defined in claim 1 for simplifying the making of a prosthetic limb socket and which significantly reduces the cost as well as the time required for making a prosthetic limb socket in addition to minimizing the equipment required for making a socket. In accordance with illustrated embodiments of the invention, a flat circular sheet or disk of thermoplastics material is injection molded in a circular mold cavity. A peripheral portion of the disk is formed or molded with one or more recesses or cavities for receiving a corresponding projection or circumferentially spaced projections of a disk support ring to form an interfitting connection between the molded disk and the support ring around the periphery of the disk to restrict lateral movement of the disk on the ring in all directions.

The preform molded plastic disk is heated on the support ring within an oven at a predetermined temperature, such as 204°C (400 degree F). After the plastics material softens and a center portion of the disk is drooping, the disk is removed from the oven on the support ring by manually gripping the support ring and an overlying peripheral portion of the disk with protective gloves. The preformed heated disk and the support ring are then inverted or flipped over as a unit and moved downwardly over a positive model of a residual limb. The softened center portion of the disk stretches around and conforms to the model while a vacuum is applied within the model through fine or small holes extending through the model to form a plastic socket conforming to the model. After the plastics material cools and becomes rigid, an annular base portion of the disk is trimmed from the socket to be discarded. After the base portion cools, it is separated from the interfitting support ring so that the ring may be reused with another injection molded disk of thermoplastics material.

Other features and advantages of the invention will be apparent from the following description, the accompanying drawings and the appended claims.

### Brief Description of the Drawings

FIG. 1 is a perspective top view of a support ring and a perspective bottom view of a molded disk and showing one form of an interfitting connection between the disk and the ring in accordance with one embodiment of the invention;
FIG. 2 is a fragmentary radial section of the interfitting ring and disk of FIG. 1 after being assembled;
FIG. 3 is a plan view of support ring and a plan view of a molded plastic disk constructed in accordance with another embodiment of the invention;
FIG. 4 is a fragmentary radial section of the interfitting plastic disk and support ring of FIG. 3 after being assembled;
FIG. 5 is a plan view of a support ring and a molded plastic disk constructed in accordance with another embodiment of the invention; and
FIG. 6 is a fragmentary radial section of the interfitting plastic disk and support ring of FIG. 5 after being assembled.

### Description of the Illustrated Embodiments

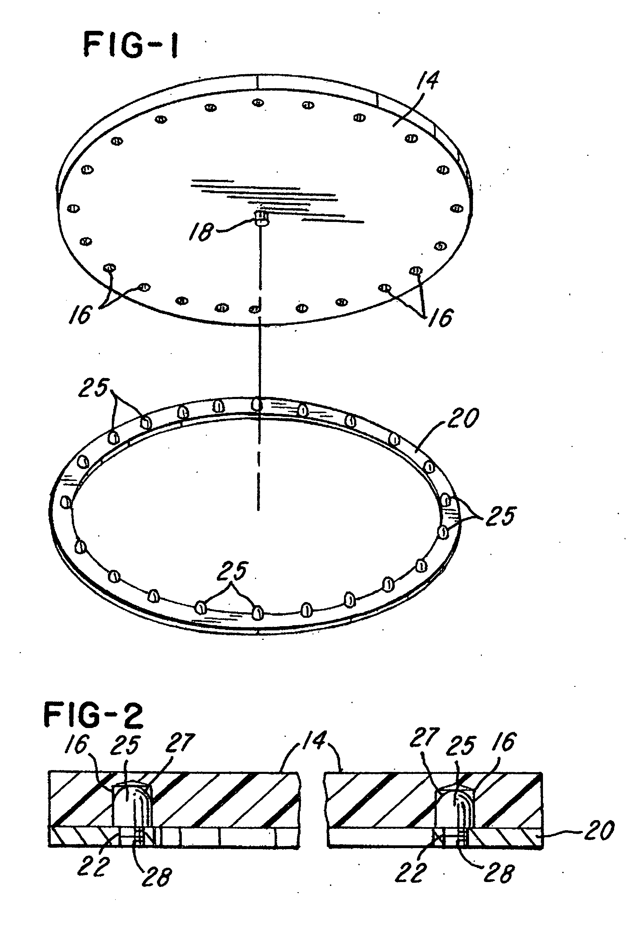

FIG. 1 illustrates a preform disk 14 which is used for making a prosthetic limb socket such as disclosed in above mentioned Patent No. 6,551,683 and which comprises a flat circular disk of a thermoplastics material such as a clear or colored polyethylene. The disk 14 has a predetermined diameter, for example, 61 cm (24 inches), and has a predetermined thickness, for example, within the range of 1,0 cm (3/8 inch) to 1,6 cm (5/8 inch), such as 1,3 cm (1/2 inch). The disk 14 is injection molded within a circular mold cavity and is molded with a series of peripherally spaced cylindrical cavities 16. Preferably each circular cavity 16 is a blind cavity in that it does not extend completely through the thickness of the disk 14. The disk is also molded with a center projection 18 which is formed by the sprue bushing supported by the mold for the disk 14.

The preform disk 14 receives and is supported by a flat metal support ring 20 which has a series of peripherally or circumferentially spaced threaded holes 22 each of which receives a projection 25 in the form of a stud having a rounded top surface 27 and a smaller diameter threaded base portion 28 threaded into a hole 22. As shown in FIG. 2, the studs or projections 25 extend into the cavities 16 molded within the disk 14 and form a positive interfitting connection between the plastic molded disk 14 and the metal support ring 20 around the ring and disk. The threaded holes 22 and the projections 25 are uniformly spaced around the support ring 20, and the holes or cavities 16 within the disk 14 are uniformly spaced around the peripheral portion of the disk 14 so that the disk 14 may be placed on the ring 20 without rotating or orienting the disk 14 to a particular location with respect to the ring 20. The projections 25 may also be attached by other means to the ring 20, for example, by welding, or projections may be formed as integral parts of the ring 20 by machining or casting the metal ring.

After the preformed disk 14 is inserted on the support ring 20, the assembled disk 14 and ring 20 are heated within an infrared or convection oven to a temperature to about 204°C (400 degrees F). After about 15 to 20 minutes, the plastics material softens, and a center portion of the disk 14 within the ring 20 begins to droop within the ring. The assembled heated disk 14 and support ring 20 are then inverted or flipped over and are moved as a unit downwardly over the positive model of the residual limb, as described in above-mentioned Patent No. 6,551,683. After the center portion of the disk 14 stretches and conforms to the positive model to form a socket with the aid of vacuum being applied through fine holes within the positive model, the socket and ring 20 are allowed to cool. An annular base portion of the socket is then trimmed from the socket, and the metal ring 20 is separated from the annular base portion so that the support ring 20 may be used again with another molded plastic preform disk 14.

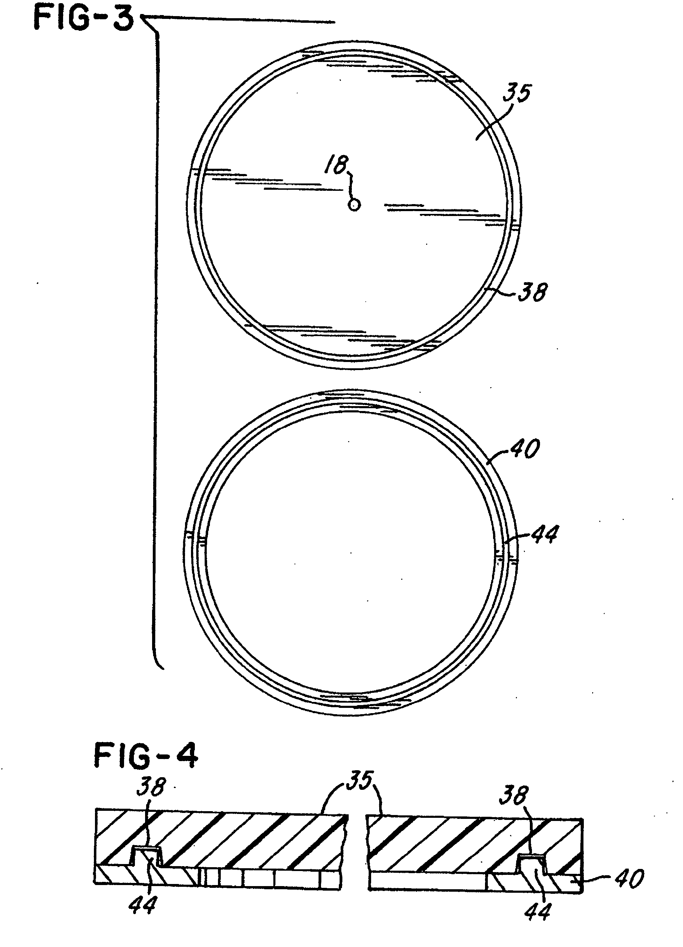

Another embodiment of the invention is illustrated in FIGS. 3 & 4. In this embodiment, a flat preform disk 35 is injection molded of a thermoplastics material and molded with an annular groove or recess 38 within a flat surface of the disk. A metal support ring 40 is formed with an annular rib or projection 44 which preferably is formed as an integral part of the support ring 40, but may be an attached annular projection. As shown in FIG. 4, when the preform disk 35 is placed on the metal support ring 40, the annular rib or projection 44 extends into the annular groove 38 to form a releasable interfitting positive connection between the disk 35 and ring 40. Preferably, the groove or recess 38 has slightly inclined or tapered side surfaces to facilitate convenient removal of an annular portion of the disk 35 from the ring 40 after forming of the prosthetic limb socket and the annular portion of the disk is cut from the socket.

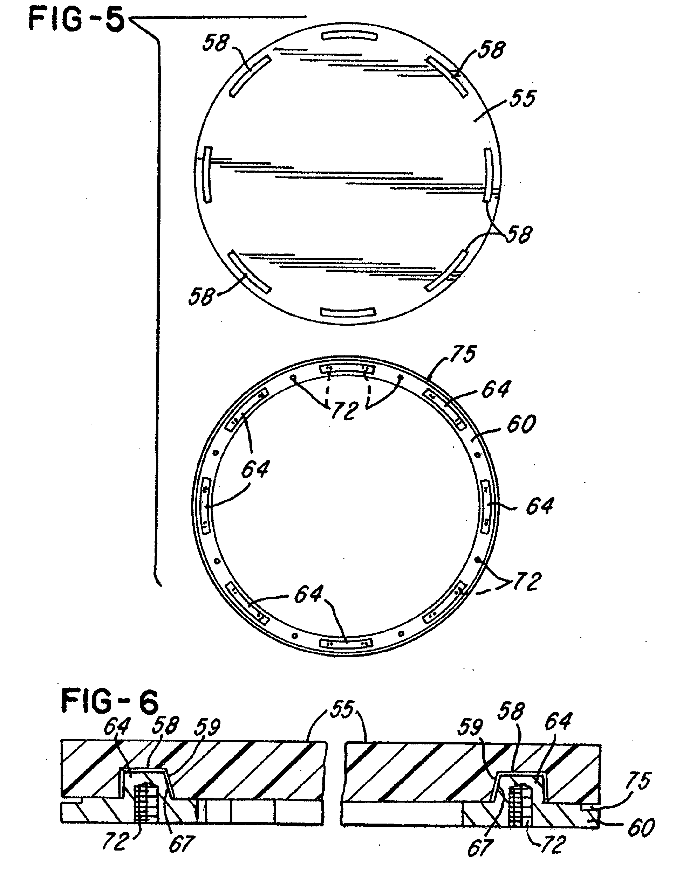

Referring to FIGS. 5 & 6 which show another modification or embodiment of the invention, a circular plastic preform disk 55 is molded of a thermoplastics material and has a peripheral portion with a plurality of circumferentially spaced arcuate grooves or recesses 58 which are uniformly spaced and project partially into the disk 55, as shown in FIG. 6. Each of the arcuate recesses 58 has a tapered or slightly inclined inner surface 59 and may have slightly tapered opposite end surfaces. A metal support ring 60 is formed with a corresponding plurality of uniformly spaced arcuate ribs or projections 64 which are preferably formed as an integral part of the ring 60, as shown in FIG. 6. Each of the arcuate projections 64 has a slightly tapered or inclined inner surface 67, and a series of threaded holes 72 are formed within the support ring 60, with two of the holes 72 extending as blind holes within each of the arcuate ribs or projections 64. The threaded holes 72 between the arcuate projections 64 extend through the support ring 60. When the preform disk 55 is placed on the support ring 60 (FIG. 6), the arcuate ribs or projections 64 extend into the arcuate recesses or cavities 58 within the preform disk 55 to form a positive interconnection of the support ring with the preform disk.

The method of using the assembled preform disk 35 and support ring 40 (FIGS. 3 & 4) and the preform disk 55 and support ring 60 (FIGS. 5 & 6) is the same as described above in connection with the preform disk 14 and the support ring 20 (FIGS. 1 & 2). After the prosthetic limb socket is formed, and the annular base portion of the preform is cut or removed from the socket, the support ring 20 or 40 or 60 is separated and removed from the annular base portion of the socket. To facilitate the separation, an annular recess 75 (FIG. 6) may be formed within the peripheral portion of the support ring 60 to form a gap with a peripheral portion of the preformed disk 55 for inserting a sharp tool, such as a screwdriver, in order to release the annular base portion of the disk from the support ring. The uniformly spaced threaded holes 72 formed within the support ring 60, as shown in FIGS. 5 & 6, may be used for receiving a series of peripherally spaced threaded studs or projections 25, as shown in FIGS, 1 & 2, in the event a molded preformed disk 14 is preferred to have circumferentially spaced holes 16 in place of arcuate recesses 58 or an annular recess 44 to form the interfitting connection.

From the drawings and the above description, it is apparent that a plastic preform for making a prosthetic limb socket and its support ring provide desirable features and advantages. For example, by molding or form ing a rigid disk of thermoplastics material and forming its metal support ring with an interfitting connection between the support ring and a peripheral portion of the disk, provides for making a prosthetic limb socket more efficiently and at a reduced cost. For example, by forming an annular recess or a series of circumferentially spaced recesses within a peripheral portion of the disk or within the ring and by forming an annular projection or a series of projections on the ring or on the disk, a disk may be simply placed on the support ring and then placed within an oven for heating.

The interfitting connection between the support ring and the plastic disk prevents a heated disk from shifting laterally relative to its support ring while the heated disk is drawn and stretched downwardly over the positive model of a residual limb by pressing downwardly on the support ring while on top of the preform disk. After the socket has been formed and trimmed to produce an annular base portion, the support ring may be quickly separated from the annular base portion of the disk, and the support ring may be reused over and over again with new preform disks. The simple and quick attachment of a disk to a support ring and the reuse of the support ring significantly reduces the time and cost for making a prosthetic limb socket.

While the forms of plastic disk and ring assembly herein described constitute preferred embodiments of the invention, it is to be understood that the invention is not limited to these precise forms, and that changes made therein without departing from the scope of the invention as defined in the appended claims.

## Claims

1. A preform for making a prosthetic limb socket from a positive model of a residual limb, comprising
an injection molded rigid disk (14,35,55) of thermoplastics material,
a rigid metal support (20,40,60) supporting a peripheral portion of said disk and having a melting temperature substantially above the melting temperature of said thermoplastics material,
said support ring having a width larger than the width of said positive model for drawing and stretching said disk downwardly over said model after said disk has been heated on said ring to a softening temperature,
said peripheral portion of said disk being separable from said support ring and after drawing and stretching said disk to permit reusing said support ring, and
**characterised by** said support ring and said peripheral portion of said disk having an interfitting connection (16,25,38,44,58,64) restricting lateral movement of said disk in any lateral direction on said support ring.

2. A preform as defined in claim 1 wherein said interfitting connection comprises a plurality of peripherally spaced cavities (16,58) molded within said peripheral portion of said disk, and said supporting ring has a corresponding plurality of peripherally spaced projections (25,64) extending into said cavities.

3. A preform as defined in claim 2 wherein each of said cavities (16) within said disk and each of said projections on said support ring is circular in cross-sectional configuration.

4. A preform as defined in claim 2 wherein each of said cavities (58) within said disk and each of said projections on said support ring and arcuate in configuration.

5. A preform as defined in claim 2 wherein said projections on said support ring are integral with said support ring.

6. A preform as defined in claim 1 wherein said interfitting connection comprises an annular cavity (38) within said peripheral portion of said disk, and said support ring includes an annular projection (44) extending into said cavity.

7. A preform as defined in claim 1 wherein said interfitting connection comprises at least one cavity within one of said disk or said support ring, the other of said disk or said support ring includes at least one projection extending into said cavity, said cavity extends only partially into said disk or said support ring, and said projection extends only into said cavity.

8. A preform as defined in claim 1 wherein said interfitting connection comprises peripherally spaced projections (25,64) on one of said support ring or said disk and circumferentially spaced cavities (16,58) within the other of said support ring or said disk.

9. A preform as defined in claim 1 wherein said peripheral portion of said disk and said support ring define a peripheral gap (75) therebetween to facilitate release of said disk from said support ring.

10. A preform as defined in claim 1 wherein said support ring has peripherally spaced threaded holes (22) extending axially into said ring, and said holes receive threaded studs (28) forming peripherally spaced projections on said support ring and threaded into said holes to form said interfitting connection.

11. A preform as defined in claim 1 wherein said interfitting connection comprises peripherally spaced projections (25,64) on a circular said support ring, and circumferentially spaced cavities (16,58) within a circular said disk.

## Patentansprüche

1. Vorform zur Herstellung eines Schafts für prothetische Gliedmaßen aus einem Positivmodell eines Stumpfs, umfassend:
eine spritzgegossene starre Scheibe (14, 35, 55) aus thermoplastischem Material,
einen starren Metallträger (20, 40, 60), der einen Randabschnitt der Scheibe trägt und eine Schmelztemperatur aufweist, die wesentlich über der Schmelztemperatur des thermoplastischen Materials liegt,
wobei der Trägerring eine Breite aufweist, die größer ist als die Breite des Positivmodells, um die Scheibe über das Modell nach unten zu ziehen und zu spannen, nachdem die Scheibe auf dem Ring auf eine Erweichungstemperatur erwärmt wurde,
wobei der Randabschnitt der Scheibe nach dem Ziehen und Spannen der Scheibe von dem Trägerring trennbar ist, damit der Trägerring wiederverwendet werden kann, und
**dadurch gekennzeichnet, dass** der Trägerring und der Randabschnitt der Scheibe eine ineinanderpassende Verbindung (16, 25, 38, 44, 58, 64) aufweisen, die die Scheibe in jeder seitlichen Richtung auf dem Trägerring in der seitlichen Bewegung einschränkt.

2. Vorform nach Anspruch 1, wobei die ineinanderpassende Verbindung eine Vielzahl von peripher beabstandeten Hohlräumen (16, 58) umfasst, die in den Randabschnitt der Scheibe geformt sind, und der Trägerring eine entsprechende Vielzahl von peripher beabstandeten Vorsprüngen (25, 64) aufweist, die sich in die Hohlräume hinein erstrecken.

3. Vorform nach Anspruch 2, wobei jeder der Hohlräume (16) in der Scheibe und jeder der Vorsprünge an dem Trägerring eine kreisförmige Querschnittsgestaltung aufweist.

4. Vorform nach Anspruch 2, wobei jeder der Hohlräume (58) in der Scheibe und jeder der Vorsprünge an dem Trägerring eine gebogene Gestalt aufweist.

5. Vorform nach Anspruch 2, wobei die Vorsprünge an dem Trägerring einteilig mit dem Trägerring sind.

6. Vorform nach Anspruch 1, wobei die ineinanderpassende Verbindung einen ringförmigen Hohlraum (38) in dem Randabschnitt der Scheibe umfasst, und der Trägerring einen ringförmigen Vorsprung (44) aufweist, der sich in den Hohlraum hinein erstreckt.

7. Vorform nach Anspruch 1, wobei die ineinanderpassende Verbindung mindestens einen Hohlraum in der Scheibe oder dem Trägerring umfasst, wobei das andere Element, Scheibe oder Trägerring, mindestens einen Vorsprung aufweist, der sich in den Hohlraum hinein erstreckt, wobei der Hohlraum sich lediglich teilweise in die Scheibe oder den Trägerring hinein erstreckt und sich der Vorsprung nur in den Hohlraum hinein erstreckt.

8. Vorform nach Anspruch 1, wobei die ineinanderpassende Verbindung peripher beabstandete Vorsprünge (25, 64) an dem Trägerring oder der Scheibe und in Umfangsrichtung beabstandete Hohlräume (16, 58) in dem anderen Element, Trägerring oder Scheibe, umfasst.

9. Vorform nach Anspruch 1, wobei der Randabschnitt der Scheibe und der Trägerring einen Randspalt (75) dazwischen definieren, damit die Scheibe leichter von dem Trägerring gelöst werden kann.

10. Vorform nach Anspruch 1, wobei der Trägerring peripher beabstandete Gewindebohrungen (22) aufweist, die axial in den Ring hinein verlaufen, und die Bohrungen Gewindebolzen (28) aufnehmen, die peripher beabstandete Vorsprünge an dem Trägerring bilden und zur Herstellung der ineinanderpassenden Verbindung in die Bohrungen hineingeschraubt sind.

11. Vorform nach Anspruch 1, wobei die ineinanderpassende Verbindung peripher beabstandete Vorsprünge (25, 64) an einem kreisförmigen derartigen Trägerring und in Umfangsrichtung beabstandete Hohlräume (16, 58) in einer kreisförmigen derartigen Scheibe umfasst.

## Revendications

1. Préforme de fabrication d'une emboîture de prothèse à partir d'un modèle positif d'un membre résiduel, comprenant :
un disque rigide moulé par injection (14, 35, 55) de matière thermoplastique,
un support métallique rigide (20, 40, 60) supportant une partie périphérique dudit disque et ayant une température de fusion sensiblement au-dessus de la température de fusion de ladite matière thermoplastique,
ladite bague de support ayant une largeur plus grande que la largeur dudit modèle positif pour étirer et étendre ledit disque vers le bas sur ledit modèle après que ledit disque a été chauffé sur ladite bague à une température de ramollissement,
ladite partie périphérique dudit disque étant séparable de ladite bague de support et après l'emboutissage et l'étirement dudit disque pour permettre la réutilisation de ladite bague de support, et
**caractérisée par le fait que** ladite bague de support et ladite partie périphérique dudit disque ont une liaison à ajustement réciproque (16, 25, 38, 44, 58, 64) limitant un mouvement latéral dudit disque dans toute direction latérale sur ladite bague de support.

2. Préforme selon la revendication 1, dans laquelle ladite liaison à ajustement réciproque comprend une pluralité de cavités espacées de façon périphérique (16, 58) moulées à l'intérieur de ladite partie périphérique dudit disque, et ladite bague de support comprend une pluralité correspondante de saillies espacées de façon périphérique (25, 64) s'étendant dans lesdites cavités.

3. Préforme selon la revendication 2, dans laquelle chacune desdites cavités (16) à l'intérieur dudit disque et chacune desdites saillies sur ladite bague de support ont une configuration circulaire en section transversale.

4. Préforme selon la revendication 2, dans laquelle chacune desdites cavités (58) à l'intérieur dudit disque et chacune desdites saillies sur ladite bague de support ont une configuration arquée.

5. Préforme selon la revendication 2, dans laquelle lesdites saillies sur ladite bague de support sont d'un seul tenant avec ladite bague de support.

6. Préforme selon la revendication 1, dans laquelle ladite liaison à ajustement réciproque comprend une cavité annulaire (38) à l'intérieur de ladite partie périphérique dudit disque, et ladite bague de support comprend une saillie annulaire (44) s'étendant dans ladite cavité.

7. Préforme selon la revendication 1, dans laquelle ladite liaison à ajustement réciproque comprend au moins une cavité à l'intérieur de l'un dudit disque ou de ladite bague de support, l'autre dudit disque ou de ladite bague de support comprend au moins une saillie s'étendant dans ladite cavité, ladite cavité s'étend uniquement partiellement dans ledit disque ou ladite bague de support, et ladite saillie s'étend uniquement dans ladite cavité.

8. Préforme selon la revendication 1, dans laquelle ladite liaison à ajustement réciproque comprend des saillies espacées de façon périphérique (25, 64) sur l'un de ladite bague de support ou dudit disque, et des cavités espacées de façon circonférentielle (16, 58) à l'intérieur de l'autre de ladite bague de support ou dudit disque.

9. Préforme selon la revendication 1, dans laquelle lesdites parties périphériques dudit disque et de ladite bague de support définissent un espace périphérique (75) entre elles pour faciliter la libération dudit disque vis-à-vis de ladite bague de support.

10. Préforme selon la revendication 1, dans laquelle ladite bague de support a des trous filetés espacés de façon périphérique (22) s'étendant axialement dans ladite bague, et lesdits trous reçoivent des goujons filetés (28) formant des saillies espacées de façon périphérique sur ladite bague de support et vissés dans lesdits trous pour former ladite liaison à ajustement réciproque.

11. Préforme selon la revendication 1, dans laquelle ladite liaison à ajustement réciproque comprend des saillies espacées de façon périphérique (25, 64) sur ladite bague de support circulaire, et des cavités espacées de façon circonférentielle (16, 58) à l'intérieur dudit disque circulaire.
